# FASCICULE DE BREVET EUROPEEN

(11) **EP 0 630 624 B1**
(45) Date de publication et mention de la délivrance du brevet: **07.01.1998**
(21) Numéro de dépôt: 94401343.2
(22) Date de dépôt: 16.06.1994
(51) Int. Cl.: A61F 2/34, A61L 27/00

(54) **Prothèse cotyloidienne, notamment pour articulation coxofémorale**
Gelenkpfannenprothese, insbesondere für Hüft-Oberschenkelgelenk
Cotyloidal prosthesis, especially for coxofemoral joint

(30) Priorité: 17.06.1993 FR 9307295
(43) Date de publication de la demande: 28.12.1994
(73) Titulaire: ROUX, Christiane, F-77123 Le Vaudoue (FR); Pequignot, Michel, F-92140 Clamart (FR)
(72) Inventeur: ROUX, Christiane, F-77123 Le Vaudoue (FR); Pequignot, Michel, F-92140 Clamart (FR)
(74) Mandataire: CABINET BONNET-THIRION

(56) Documents cités:
- EP-A- 0 051 686
- EP-A- 0 053 794
- EP-A- 0 226 762
- EP-A- 0 315 795
- DE-U- 9 215 863
- FR-A- 2 225 141
- FR-A- 2 626 766
- FR-A- 2 635 968
- FR-A- 2 699 067

## Description

La présente invention concerne d'une manière générale les prothèses cotyloïdiennes, et elle vise plus particulièrement, mais non nécessairement exclusivement, le cas où une telle prothèse cotyloïdienne est destinée à la reconstitution d'une articulation coxo-fémorale.

Ainsi qu'on le sait, une articulation coxo-fémorale prothétique met globalement en oeuvre deux pièces complémentaires, à savoir, d'une part, une cuvette hémisphérique, ou cotyle, qui est destinée à être fixée, soit directement, soit indirectement, par l'intermédiaire d'une pièce de fixation, à la cavité cotyloïde de l'os iliaque du sujet à traiter, et, d'autre part, une tête sphérique, en forme de bille, présente à l'extrémité d'une tige, qui elle, est destinée à être enfoncée dans le canal fémoral de celui-ci.

Compte tenu, d'une part, de l'excellent coefficient de frottement que présente une céramique, et notamment une céramique d'alumine, et compte tenu, d'autre part, de la biocompatibilité d'un tel matériau avec les tissus osseux, il a déjà été proposé et effectué de réaliser en céramique tant la tête sphérique que la cuvette.

Cela est le cas, par exemple, dans la demande de brevet français No 2 225 141.

Même si, s'agissant de la tête sphérique, l'usinage d'une bille en céramique est relativement simple, et par conséquent, d'un coût relativement acceptable, il n'en est pas de même pour la cuvette.

En pratique, eu égard, notamment, à la précision requise pour l'obtention d'une parfaite congruence avec la tête sphérique associée, et eu égard, également, au fait qu'il s'agit à ce jour d'une pièce relativement massive réalisée d'un seul tenant, le coût d'une cuvette en céramique est particulièrement élevé.

En effet, la précision d'usinage requise nécessite, en pratique, d'avoir recours, pour cette cuvette, aux techniques utilisées en optique, en l'espèce un usinage par rodage effectué avec des abrasifs de plus en plus fins.

La tête sphérique et la cuvette donnent lieu ensuite à un usinage terminal par rodage mutuel, ce qui nécessite de procéder à un appariement précis de ces pièces, avec les difficultés considérables que cela implique au stockage et à l'emploi.

Comme, par ailleurs, il est souvent constaté, en particulier chez les sujets âgés, qui sont majoritairement concernés par les articulations coxo-fémorales prothétiques, un descellement à terme de la cuvette lorsqu'elle est réalisée en céramique, ce descellement pouvant par exemple être attribué dans ce cas à un mauvais accrochage de la céramique aux tissus osseux, les cuvettes en céramique ne sont le plus souvent utilisées à ce jour que pour les seuls sujets jeunes, les tissus osseux de ceux-ci paraissant s'accommoder mieux d'un tel matériau, vraisemblablement parce que l'amortissement des chocs normaux tels que ceux dus à la marche est meilleur chez eux.

Chez les sujets âgés, la cuvette est donc le plus souvent à ce jour en matière synthétique, et plus précisément, en polyéthylène haute densité, cependant que, préférentiellement, la tête sphérique reste, elle, en céramique.

Mais, outre une production inévitable de débris de polyéthylène accusés à l'heure actuelle de pouvoir provoquer des dégâts au niveau osseux, les articulations coxo-fémorales prothétiques comportant ainsi une cuvette en matière synthétique ont pour inconvénient une longévité moindre que celles dans lesquelles cette cuvette est en céramique.

Il s'avère, en effet, suivant divers travaux que, progressivement, et quelle que soit sa matière constitutive, la tête sphérique s'enfonce verticalement dans la cuvette, en partie à cause du fluage de la matière synthétique de celle-ci et en partie à cause de l'usure de cette matière synthétique sous les effets de la tête sphérique.

C'est d'ailleurs la raison pour laquelle, une longévité améliorée étant recherchée pour les sujets jeunes, les articulations coxo-fémorales prothétiques mettant en oeuvre un couple de frottement céramique-céramique, c'est-à-dire celles dans lesquelles tant la cuvette que la tête sphérique sont en céramique, connaissent aujourd'hui un regain d'intérêt.

Dans ce cas, en effet, le fluage est nul et l'usure quasi négligeable.

Tout risque de production de débris en polyéthylène est en outre ainsi évité.

La présente invention a d'une manière générale pour objet une prothèse cotyloïdienne, qui, par des perfectionnements à la mise en oeuvre d'un couple de frottement céramique-céramique, assure de manière très simple un compromis avantageux entre les articulations coxo-fémorales prothétiques à cuvette en céramique et celles à cuvette en matière synthétique, et qui présente en outre d'autres avantages.

Elle est fondée sur l'observation que, dans la station bipède, et tel que cela résulte notamment de travaux de Friedrich PAUWELS remontant à 1965, les parties effectivement sollicitées des constituants osseux concernés, à savoir la cavité cotyloïde et la tête fémorale, se trouvent en pratique le plus communément limitées à une portion réduite du croissant articulaire, correspondant sensiblement au seul toit de la cavité cotyloïde.

La pesanteur et la fonction locomotrice font en effet que la pression de la tête fémorale sur la cavité cotyloïde est la plus importante au niveau de ce toit.

Cela étant, la prothèse cotyloïdienne suivant l'invention, qui convient notamment à une articulation coxo-fémorale, et qui est du genre comportant, pour coopération avec une tête sphérique, une pièce en céramique dont la surface interne appartient à une sphère, est d'une manière générale caractérisée en ce que ladite pièce en céramique se réduit à un anneau, et en ce que l'anneau formant ainsi cette pièce en céramique est fretté par une virole.

Ainsi, suivant l'invention, il est assuré une répartition des fonctions entre, d'une part, une partie interne, en céramique, en l'espèce l'anneau, qui a pour charge de coopérer en articulation avec la tête sphérique, et d'autre part, une partie externe, par exemple une cuvette en matière synthétique, qui n'a pour charge que d'assurer la liaison de l'anneau avec la cavité cotyloïde, en étant de manière usuelle dûment fixée à celle-ci, soit directement, par exemple par cimentation, soit indirectement, par exemple par l'intermédiaire d'une pièce de fixation métallique.

Mais, en limitant à la portion utile, c'est-à-dire fonctionnelle, la partie en céramique, tous les avantages dus à la mise en oeuvre de céramique se trouvent conservés tout en en réduisant singulièrement le coût.

En effet, en ne s'étendant que sur une fraction de la profondeur de la cuvette en matière synthétique, l'anneau en céramique mis en oeuvre suivant l'invention a une hauteur et une épaisseur qui sont singulièrement réduites par rapport à celles de cette cuvette.

Ainsi la quantité de céramique à mettre en oeuvre se trouve diminuée dans de fortes proportions, en étant par exemple de 3 à 6 fois moindre.

En outre, le coût de l'usinage et du polissage de l'anneau se trouve lui-même réduit par rapport à celui d'une cuvette.

Par ailleurs, lorsque, par exemple, une pièce de fixation est mise en oeuvre, la présence de l'anneau en céramique permet avantageusement de ménager, avec toute l'amplitude nécessaire, à la surface extérieure de la cuvette en matière synthétique, les évidements propres au logement des moyens de fixation devant assurer l'assujettissement de cette pièce de fixation à la cavité cotyloïde, sans risque que l'amincissement local qui en résulte pour cette cuvette aux endroits correspondants ne soit susceptible de conduire, après fluage et usure, à un frottement direct de la tête sphérique avec ces moyens de fixation, comme cela pourrait être le cas en l'absence d'un tel anneau.

Conjointement, l'anneau en céramique mis en oeuvre suivant l'invention est fretté extérieurement par une virole, ce qui en améliore avantageusement la résistance mécanique, et, en particulier, la résistance aux chocs et à l'éclatement.

Ainsi qu'on le sait, les contraintes radiales dues au frettage s'opposent efficacement à la propagation des microfissures, telles que criques, pouvant apparaître dans les céramiques, notamment sous l'effet de contraintes alternées et/ou en présence d'un liquide de mouillage, comme cela est le cas dans une articulation prothétique.

Eu égard à ses dimensions relativement réduites, il peut parallèlement être envisagé de réaliser à partir d'un mono-cristal, taillé dans l'axe voulu, l'anneau en céramique, au lieu de le réaliser par frittage, ce qui éliminerait radicalement tout risque de déchaussement d'un quelconque grain, avec les conséquences d'un tel déchaussement, inhérent à une telle réalisation par frittage.

Quoi qu'il en soit, eu égard, encore, à son épaisseur réduite, il est aussi possible d'envisager, à encombrement hors tout égal, d'entourer par une bague en matière élastique l'anneau en céramique, pour améliorer les conditions d'amortissement de l'ensemble de la prothèse.

Les caractéristiques et avantages de l'invention ressortiront d'ailleurs de la description qui va suivre, à titre d'exemple, en référence aux dessins schématiques annexés sur lesquels :
la figure 1 est une vue en perspective d'une prothèse cotyloïdienne suivant l'invention ;
la figure 2 en est, à échelle supérieure, une vue en coupe axiale, suivant la ligne II-II de la figure 1 ;
la figure 3 reprend, à échelle supérieure, le détail de la figure 2 repéré par un encart III sur cette figure 2 ;
les figures 5 et 6, sont des vues partielles en coupe axiale, qui, correspondant à celle de la figure 3, se rapportent chacune respectivement à diverses formes de réalisation ;
la figure 7 est une vue en coupe axiale, qui, analogue, elle aussi, à celle de la figure 2, se rapporte à une autre forme de réalisation;

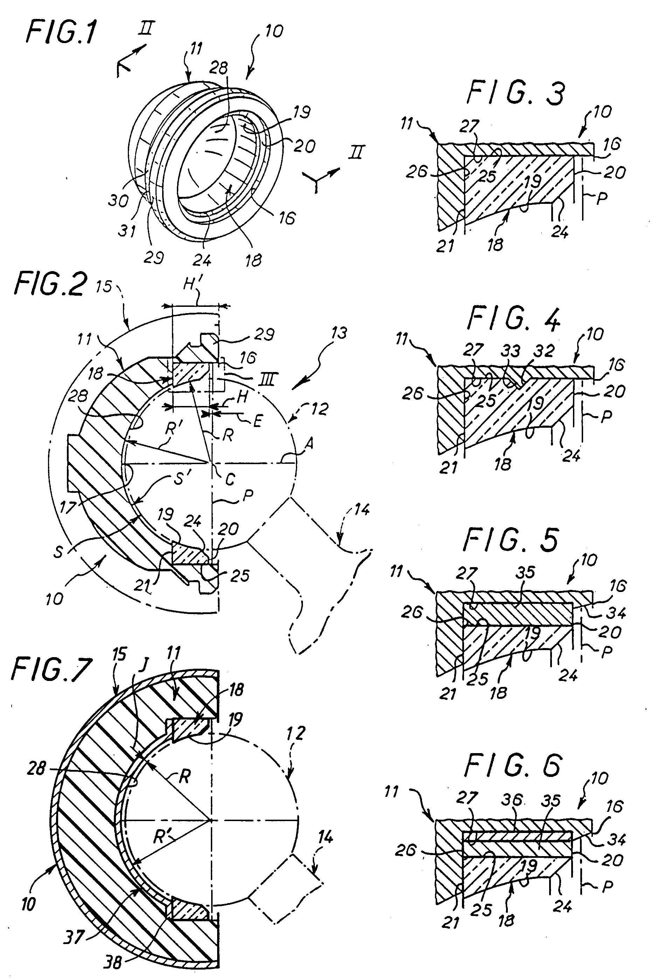

La figure 4 est une vue en coupe axiale réalisée à des fins d'illustration et qui ne constitue pas un exemple de réalisation conformément à l'invention.

Tel qu'illustré sur ces figures, la prothèse cotyloïdienne 10 suivant l'invention comporte, de manière connue en soi, une cuvette 11 en matière synthétique, et, destinée à être implantée, extérieurement, dans une cavité cotyloïde, non représentée, elle est adaptée à coopérer, intérieurement, avec une tête sphérique 12 tel que schématisé en traits interrompus sur la figure 2.

L'articulation prothétique 13 que forme l'ensemble reconstitue, par exemple, et tel que représenté, une articulation coxo-fémorale.

La cavité cotyloïde concernée appartient alors à l'os iliaque du sujet traité, cependant que la tête sphérique 12 est présente au bout d'une tige 14, dûment engagée et solidarisée dans le canal fémoral de celui-ci.

Par exemple, cette tête sphérique 12 est une bille en céramique, et, par exemple, en céramique d'alumine, elle est dûment rapportée, par exemple par emmanchement conique, sur la tige 14, et cette tige 14 est par exemple en métal.

Par exemple, également, la cuvette 11 est en polyéthylène haute densité, et, pour son assujettissement à la cavité cotyloïde, il lui est associé, extérieurement, tel que schématisé en traits interrompus sur la figure 2, une pièce de fixation 15 qui est elle-même globalement en forme de cuvette, et dans laquelle elle se trouve par exemple rapportée par encliquetage.

Par exemple, cette pièce de fixation 15 est métallique.

De manière usuelle, sa solidarisation à la cavité cotyloïde peut par exemple être assurée par vissage, impactage ou cimentation.

Les dispositions précédentes étant globalement bien connues par elles-mêmes, et ne relevant pas de la présente invention, elles ne seront pas décrites plus en détail ici.

De manière également connue en soi, la prothèse cotyloïdienne 10 comporte, pour coopération avec la tête sphérique 12, une pièce en céramique 18.

Suivant l'invention, cette pièce en céramique 18 se réduit à un anneau dont la surface interne 19 appartient à une sphère S.

Bien entendu, le rayon R de cette sphère S est égal à celui de la tête sphérique 12.

Autrement dit, la sphère S est complémentaire de la tête sphérique 12.

Soit C le centre de cette sphère S.

Dans les formes de réalisation représentées, l'anneau formant la pièce en céramique 18 est porté par la cuvette 11.

Il est disposé dans cette cuvette 11, au voisinage du débouché de celle-ci.

Préférentiellement, la hauteur H de l'anneau formant la pièce en céramique 18, mesurée parallèlement à son axe A, est au plus égale à la moitié du rayon R de la sphère S.

Par exemple, cette hauteur H est comprise entre le tiers et la moitié du rayon R.

En toute hypothèse, l'anneau formant la pièce en céramique 18 ne s'étend que sur une fraction de la profondeur de la cuvette 11, mesurée entre son débouché 16 et son fond 17.

Dans les formes de réalisation représentées, les faces avant 20 et arrière 21 de l'anneau formant la pièce en céramique 18 s'étendent suivant deux plans parallèles, perpendiculairement à l'axe A.

Plus précisément, dans ces formes de réalisation, la face avant 20 de l'anneau formant la pièce en céramique 18 s'étend suivant un plan qui est légèrement en retrait par rapport au plan équatorial P de la sphère S perpendiculaire à l'axe A, en se situant entre ce plan équatorial P et le fond 17 de la cuvette 11.

En pratique, l'écart E correspondant est faible.

Pour être plus apparent, il a été volontairement exagéré sur les figures.

Dans les formes de réalisation représentées, l'arête de l'anneau formant la pièce en céramique 18 correspondant au raccordement de sa surface interne 19 avec sa face avant 20 est abattue par un chanfrein 24, pour en éviter la fragilité.

La surface de frottement correspondante s'en trouve corollairement réduite d'autant.

Sur les figures, le chanfrein 24 a été volontairement exagéré.

Dans les formes de la réalisation représentées, enfin, la surface externe 25 de l'anneau formant la pièce en céramique 18 est cylindrique, avec des génératrices parallèles à l'axe A.

L'anneau formant la pièce en céramique 18 ainsi constituée est préférentiellement en céramique d'alumine.

Suivant des techniques communes en la matière, il est par exemple réalisé par frittage, avec compression isostatique ou traitement H.I.P. ("High Isostatic Pressure").

Par exemple, et c'est le cas dans la forme de réalisation plus particulièrement représentée sur les figures 1 à 3, l'anneau formant la pièce en céramique 18 est simplement engagé à force par sa surface externe 25 dans la cuvette 11.

Cette cuvette 11 présente donc, intérieurement, pour ce faire, à compter de son débouché 16, et jusqu'à un épaulement transversal 26, une portée cylindrique 27.

Dans les formes de réalisation représentées, cette portée cylindrique 27 à une hauteur H' légèrement supérieure à celle H de l'anneau formant la pièce en céramique 18 en sorte que la face avant 20 de celui-ci est en retrait par rapport au débouché 16 de la cuvette 11.

A compter de l'anneau formant la pièce en céramique 18, et, donc, à compter de l'épaulement transversal 26, la surface interne 28 de la cuvette 11 s'étend préférentiellement à l'écart de la sphère S à laquelle appartient la surface interne 19 de l'anneau formant la pièce en céramique 18.

Dans les formes de réalisation représentées, la surface interne 28 de la cuvette 11 appartient elle aussi à une sphère S', concentrique de la sphère S, et le diamètre R' de cette sphère S' est supérieur à celui R de la sphère S.

Extérieurement, la cuvette 11 présente, annulairement au droit de son débouché 16, dans les formes de réalisation représentées, un rebord 29.

Dans les variantes de réalisation représentées, sur les figures 5 et 6, il est prévu, entre la cuvette 11 et l'anneau formant la pièce en céramique 18, des moyens d'encliquetage.

Dans la figure 4, qui ne constitue pas un exemple de réalisation conforme à la présente invention, l'anneau formant la pièce en céramique 18 présente, annulairement en creux sur sa surface externe 25, une gorge 32, et, de manière complémentaire, la cuvette 11 présente, annulairement en saillie sur sa portée cylindrique 27, un cordon 33.

Dans l'exemple représenté à la figure 4, la gorge 32 et le cordon 33 ont en section transversale un profil triangulaire.

En variante, figures 5 et 6, la cuvette 11 comprend, annulairement en saillie sur sa portée cylindrique 27 à compter de son débouché 16, pour l'encliquetage de l'anneau formant la pièce en céramique 18, un bourrelet d'engagement 34 à surface extérieure tronconique.

Suivant l'invention, et tel que représenté sur les figures 5 et 6, l'anneau formant la pièce en céramique 18 est fretté extérieurement sur toute sa hauteur par une virole 35, posée à chaud.

Cette virole 35 est en pratique métallique.

Elle est par exemple en titane allié, en acier inoxydable à bas taux de carbone ou en alliage de type stellite, tous matériaux qui sont avantageusement biocompatibles.

Dans la variante de réalisation représentée sur la figure 6, l'anneau formant la pièce en céramique 18 est en outre, entouré, sur toute sa hauteur, par une bague 36 qui, réalisée en matière élastique, et, par exemple, en élastomère, intervient radialement entre lui et la cuvette 11.

Dans la variante de réalisation représentée sur la figure 7, la cuvette 11 en matière synthétique est doublée intérieurement par une cupule 37, en métal, qui, destinée à s'opposer à tout fluage de sa part, s'étend préférentiellement à son contact en chacun de ses points.

Par exemple, cette cupule 37 est en titane, et son épaisseur est limitée à quelques dixièmes de millimètre.

Dans la forme de réalisation représentée, elle présente, radialement en saillie vers l'extérieur le long de son bord libre, une collerette 38, qui est engagée sous la pièce en céramique 18, entre celle-ci et l'épaulement correspondant de la cuvette 11 en matière synthétique.

Pour le reste, les dispositions sont du type de celles décrites précédemment.

En particulier, il subsiste, radialement, en service, un jeu J entre la tête sphérique 12 et la cupule 37.

Autrement dit, la surface interne 28 de la cuvette 11 doit dans ce cas être considérée comme formée par la surface interne de la cupule 37.

Bien entendu, la présente invention ne se limite pas aux formes de réalisation décrites et représentées, mais englobe toute variante d'exécution et/ou de combinaison de leurs divers éléments.

En outre, la céramique susceptible d'être mise en oeuvre n'est pas nécessairement la seule céramique d'alumine plus particulièrement mentionnée.

Il peut au contraire s'agir tout aussi bien de céramique de zircone, de céramique de nitrure de silicium, ou, d'une manière plus générale, de céramique de tout matériau susceptible d'être fritté à haute température.

## Revendications

1. Prothèse cotyloïdienne, notamment pour articulation coxo-fémorale, du genre comportant pour coopération avec une tête sphérique (12), une pièce (18) en céramique dont la surface interne (19) appartient à une sphère (S), caractérisée en ce que la pièce en céramique (18) se réduit à un anneau, et en ce que l'anneau formant ainsi cette pièce en céramique (18) est fretté par une virole (35).

2. Prothèse cotyloïdienne suivant la revendication 1, caractérisée en ce que la hauteur (H) de l'anneau formant la pièce en céramique (18), mesurée parallèlement à son axe (A), est au plus égale à la moitié du rayon (R) de la sphère (S).

3. Prothèse cotyloïdienne suivant la revendication 2, caractérisée en ce que la hauteur (H) de l'anneau formant la pièce en céramique (18) est comprise entre le tiers et la moitié du rayon (R) de la sphère (S).

4. Prothèse cotyloïdienne suivant l'une quelconque des revendications 1 à 3, caractérisée en ce que les faces avant (20) et arrière (21) de l'anneau formant la pièce en céramique (18) s'étendent suivant deux plans parallèles.

5. Prothèse cotyloïdienne suivant l'une quelconque des revendications 1 à 4, caractérisée en ce que la face avant (20) de l'anneau formant la pièce en céramique (18) s'étend suivant un plan qui est légèrement en retrait par rapport à un plan équatorial (P) de la sphère (S).

6. Prothèse cotyloïdienne suivant l'une quelconque des revendications 1 à 5, caractérisée en ce que la surface externe (25) de l'anneau formant la pièce en céramique (18) est cylindrique.

7. Prothèse suivant l'une quelconque des revendications 1 à 6, caractérisée en ce que la virole (35) est métallique.

8. Prothèse cotyloïdienne suivant l'une quelconque des revendications 1 à 7, caractérisée en ce que l'anneau formant la pièce en céramique (18) est porté par une cuvette (11) en matière synthétique, en étant disposé dans celle-ci, au voisinage de son débouché (16).

9. Prothèse cotyloïdienne suivant la revendication 8, caractérisée en ce que la cuvette (11) en matière synthétique est doublée intérieurement par une cupule (37) en métal.

10. Prothèse cotyloïdienne suivant l'une quelconque des revendications 8, 9, caractérisée en ce que, à compter de l'anneau formant la pièce en céramique (18), la surface interne (28) de la cuvette (11) s'étend à l'écart de la sphère (S) à laquelle appartient la surface interne (19) de celui-ci.

11. Prothèse cotyloïdienne suivant la revendication 10, caractérisée en ce que la surface interne (28) de la cuvette (11) appartient à une sphère (S') de rayon (R') supérieur à celui (R) de la sphère (S) à laquelle appartient la surface interne (19) de l'anneau formant la pièce en céramique (18).

12. Prothèse cotyloïdienne suivant l'une quelconque des revendications 8 à 11, caractérisée en ce que, entre l'anneau formant la pièce en céramique (18) et la cuvette (11), intervient radialement une bague (36) en matière élastique.

13. Prothèse cotyloïdienne suivant l'une quelconque des revendications 8 à 12, caractérisée en ce que l'anneau formant la pièce en céramique (18) est engagé à force dans la cuvette (11).

14. Prothèse cotyloïdienne suivant l'une quelconque des revendications 8 à 13, caractérisée en ce que, entre la cuvette (11) et l'anneau formant la pièce en céramique (18), il est prévu des moyens d'encliquetage.

15. Prothèse cotyloïdienne suivant l'une quelconque des revendications 1 à 14, caractérisée en ce qu'il lui est associé extérieurement une pièce de fixation (15).

## Claims

1. A cotyloidal prosthesis, in particular for a coxofemoral joint, of the kind comprising for co-operation with a spherical head (12) a ceramic part (18) whose internal surface (19) belongs to a sphere (S), characterised in that the ceramic part (18) is reduced to a ring and that the ring forming said ceramic part (18) is hooped by a band (35).

2. A cotyloidal prosthesis according to claim 1 characterised in that the height (H) of the ring forming the ceramic part (18), as measured parallel to its axis (A), is at most equal to half the radius (R) of the sphere (S).

3. A cotyloidal prosthesis according to claim 2 characterised in that the height (H) of the ring forming the ceramic part (18) is between a third and half the radius (R) of the sphere (S).

4. A cotyloidal prosthesis according to any one of claims 1 to 3 characterised in that the front and rear faces (20, 21) of the ring forming the ceramic part (18) extend in two parallel planes.

5. A cotyloidal prosthesis according to any one of claims 1 to 4 characterised in that the front face (20) of the ring forming the ceramic part (18) extends in a plane which is slightly set back with respect to an equatorial plane (P) of the sphere (S).

6. A cotyloidal prosthesis according to any one of claims 1 to 5 characterised in that the outside surface (25) of the ring forming the ceramic part (18) is cylindrical.

7. A cotyloidal prosthesis according to any one of claims 1 to 6 characterised in that the band (35) is metal.

8. A cotyloidal prosthesis according to any one of claims 1 to 7 characterised in that the ring forming the ceramic part (18) is carried by a cup (11) of synthetic material, being disposed in same in the proximity of its opening (16).

9. A cotyloidal prosthesis according to claim 8 characterised in that the cup (11) of synthetic material is internally lined by a metal cup (37).

10. A cotyloidal prosthesis according to either one of claims 8 and 9 characterised in that starting from the ring forming the ceramic part (18), the inside surface (28) of the cup (11) extends at a spacing from the sphere (S) to which the inside surface (19) of same belongs.

11. A cotyloidal prosthesis according to claim 10 characterised in that the inside surface (28) of the cup (11) belongs to a sphere (S') whose radius (R') is greater than that (R) of the sphere (S) to which belongs the inside surface (19) of the ring forming the ceramic part (18).

12. A cotyloidal prosthesis according to any one of claims 8 to 11 characterised in that a ring portion (36) of elastic material is radially disposed between the ring forming the ceramic part (18) and the cup (11).

13. A cotyloidal prosthesis according to any one of claims 8 to 12 characterised in that the ring forming the ceramic part (18) is a force fit in the cup (11).

14. A cotyloidal prosthesis according to any one of claims 8 to 13 characterised in that latching means are provided between the cup (11) and the ring forming the ceramic part (18).

15. A cotyloidal prosthesis according to any one of claims 1 to 14 characterised in that a fixing member (15) is externally associated therewith.

## Patentansprüche

1. Gelenkpfannenprothese, insbesondere für ein Hüft-Oberschenkelgelenk, welche für ein Zusammenwirken mit einem kugelförmigen Kopf (12) ein Teil (18) aus Keramik umfaßt, dessen Innenfläche (19) zu einer Kugel (S) gehört, dadurch gekennzeichnet, daß das Keramikteil (18) auf einen Ring reduziert ist und daß der derart das Keramikteil (18) bildende Ring von einer Einfassung bzw. einem Ring (35) umgeben ist.

2. Gelenkpfannenprothese nach Anspruch 1, dadurch gekennzeichnet, daß die Höhe (H) des das Keramikteil (18) bildenden Ringes, parallel zu seiner Achse (A) gemessen, höchstens gleich der Hälfte des Radius (R) der Kugel (S) ist.

3. Gelenkpfannenprothese nach Anspruch 2, dadurch gekennzeichnet, daß die Höhe (H) des das Keramikteil (18) bildenden Ringes zwischen einem Drittel und der Hälfte des Radius (R) der Kugel (S) liegt.

4. Glenkpfannenprothese nach einem der Ansprüche 1 bis 3, dadurch gekennzeichnet, daß sich die vordere (20) und hintere (21) Fläche des das Keramikteil (18) bildenden Ringes längs zweier paralleler Ebenen erstrecken.

5. Glenkpfannenprothese nach einem der Ansprüche 1 bis 4, dadurch gekennzeichnet, daß sich die vordere Fläche (20) des das Keramikteil (18) bildenden Ringes längs einer Ebene erstreckt, welche geringfügig relativ zu einer Äquatorebene (P) der Kugel (S) rückversetzt ist.

6. Glenkpfannenprothese nach einem der Ansprüche 1 bis 5, dadurch gekennzeichnet, daß die äußere Oberfläche (25) des das Keramikteil (18) bildenden Ringes zylindrisch ist.

7. Prothese nach einem der Ansprüche 1 bis 6, dadurch gekennzeichnet, daß die Einfassung (35) metallisch ist.

8. Gelenkpfannenprothese nach einem der Ansprüche 1 bis 7, dadurch gekennzeichnet, daß der das Keramikteil (18) bildende Ring von einer Schale (11) aus Kunststoff getragen ist und in dieser nahe deren Öffnung (16) angeordnet ist.

9. Glenkpfannenprothese nach Anspruch 8, dadurch gekennzeichnet, daß die Schale (11) aus Kunststoff innen durch eine Kuppel (37) aus Metall ausgefüttert ist.

10. Gelenkpfannenprothese nach einem der Ansprüche 8 oder 9, dadurch gekennzeichnet, daß ausgehend von dem das Keramikteil (18) bildenden Ring die Innenfläche (28) der Schale (11) sich in Abstand von der Kugel (S) erstreckt, an welche sich die Innenfläche (19) desselben annähert.

11. Glenkpfannenprothese nach Anspruch 10, dadurch gekennzeichnet, daß die Innenfläche (28) der Schale (11) zu einer Kugel (S') mit einem Radius (R') gehört, welcher größer ist als jener (R) der Kugel (S), zu welcher die Innenfläche (19) des das Keramikteil (18) bildenden Ringes gehört.

12. Gelenkpfannenprothese nach einem der Ansprüche 8 bis 11, dadurch gekennzeichnet, daß zwischen dem das Keramikteil (18) bildenden Ring und der Schale (11) in radialer Richtung ein Ring (36) aus Kunststoff zwischengeschaltet ist.

13. Gelenkpfannenprothese nach einem der Ansprüche 8 bis 12, dadurch gekennzeichnet, daß der das Keramikteil (18) bildenden Ring kraftschlüssig in der Schale (11) in Eingriff steht.

14. Glenkpfannenprothese nach einem der Ansprüche 8 bis 13, dadurch gekennzeichnet, daß zwischen der Schale (11) und dem das Keramikteil (18) bildenden Ring Einrastvorrichtungen vorgesehen sind.

15. Glenkpfannenprothese nach einem der Ansprüche 1 bis 14, dadurch gekennzeichnet, daß dieser außen ein Befestigungsstück (15) zugeordnet ist.
